(19) Europäisches Patentamt / European Patent Office / Office européen des brevets

(11) **EP 3 157 626 B1**

(12) **EUROPEAN PATENT SPECIFICATION**

(45) Date of publication and mention of the grant of the patent:
**07.11.2018 Bulletin 2018/45**

(21) Application number: **15727426.7**

(22) Date of filing: **11.06.2015**

(51) Int Cl.:
***A61N 5/10*** *(2006.01)*

(86) International application number:
**PCT/EP2015/063008**

(87) International publication number:
**WO 2015/193162 (23.12.2015 Gazette 2015/51)**

(54) **SYSTEM AND COMPUTER PROGRAM PRODUCT FOR RADIATION INVERSE TREATMENT PLANNING**

SYSTEM UND COMPUTERPROGRAMMPRODUKT FÜR INVERSE BESTRAHLUNGSBEHANDLUNGSPLANUNG

SYSTÈME ET PRODUIT PROGRAMME D'ORDINATEUR POUR LA PLANIFICATION DE TRAITEMENT INVERSE DE RAYONNEMENT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.06.2014 CH 915142014**
**17.06.2014 CH 914142014**

(43) Date of publication of application:
**26.04.2017 Bulletin 2017/17**

(73) Proprietor: **Intuitive Therapeutics SA**
**1025 St-Sulpice (CH)**

(72) Inventors:
• **MARTIN, André**
**CH-1028 Préverenges (CH)**

• **SALZMANN, Daniel**
**CH-1162 Saint-Prex (CH)**

(74) Representative: **P&TS SA (AG, Ltd.)**
**Av. J.-J. Rousseau 4**
**P.O. Box 2848**
**2001 Neuchâtel (CH)**

(56) References cited:
**US-A1- 2011 085 643     US-A1- 2012 136 194**

• **SVANTE SÖDERSTRÖM ET AL: "WHICH IS THE MOST SUITABLE NUMBER OF PHOTON BEAM PORTALS IN COPLANAR RADIATION THERAPY?", INT. J. RADIATION ONCOLOGY BIOL. PHYS, vol. 33, no. 1, 1 January 1995 (1995-01-01), pages 151-159, XP055202759, DOI: 0360-3016(95)00113-1**

## Description

Field of the invention

Embodiments of the present invention relate to a system for the planning and real-time controlling of radiation treatments. Without loss of generality, the system of the present invention can be usefully employed for planning treatments of radiotherapy by collimated radiation sources of various nature like, for example multi-beam gamma irradiations, photon beams generated by linear accelerators, conventional high-voltage X-ray sources, or radioactive sources. The invention can usefully be employed also for planning and controlling therapeutic irradiations by charged beams, for example electrons, ions, or hadrons.

Description of related art

Many radiation therapy systems, including radiotherapy and radiosurgery systems, use γ-radioactive isotopes or particle accelerators (typically linear electron accelerators, known in short as LINACs) which produce a single radiation beam, to irradiate a target region of the body. Even if photon therapy is prevalent, irradiation by particle beams, for example electrons and protons, are also used and have shown a very high success rate in their respective application areas. The same difficulties of selective irradiation of non-static structures are present as in the case of photon therapy, and the present invention can be usefully applied to any form of radiation therapy.

In an effort to control the dose deposition patter in order to achieve an optimal therapeutic effect and reduce, as much as possible the dose delivered to healthy tissues, several types of collimators and irradiation geometries have been developed, often with considerable success.

In LINAC-based radiotherapy systems, the irradiation is not performed not only under one single incidence (i.e. one shot), corresponding to a fixed pre-determined position and orientation of the radiation beam with respect to the target, but it uses multiple successive incidences to increase the conformity of the dose delivery. A large number of multiple incidences is used to perform the so-called Linac-based radiosurgery.

Beam cross-sections are typically comprised in the range 10 cm - 30 cm, e.g. 20 cm in the former case, and may be of 4, 8, or 16 mm, depending on the size of collimators selected, in the latter. LINAC-based radiation therapy are usually equipped with collimator devices for modifying the transverse beam dimensions according to the selected treatment plan. Several recent radiotherapy and radiosurgery systems include multi-leaf and micro-multi-leaf automatic collimators that can be controlled during the treatment to produce arbitrary-shaped radiation beams with a geometric precision in the millimetre range.

In most instances, the irradiation of a linear accelerator is performed not under one single incidence (i.e. one shot) corresponding to a fixed pre-determined position and orientation of the radiation beam with respect to the target, but according to multiple successive incidences to increase the conformity of the dose delivery. A large number of multiple incidences is used to perform the so-called LINAC-based radiosurgery.

In the context of the present invention, the noun "weight" refers to one or more parameters of the shot, e.g. in a non-limiting way, the time of irradiation and/or the dose rate and/or the dose profile, etc.

In most of the LINAC-based radiotherapy systems, the emission head is attached to a physical support (called the "gantry") that can be mechanically rotated around the patient, in a full or partial circle. The table where the patient is lying (called the "couch"), can sometimes also be moved in linear or angular steps. The intensity and shape of the radiation beams are adapted on an incidence-by-incidence basis, or possibly even within a single incidence, by suitably controlling the LINAC and by automatic multi-leaf collimators.

The combination of the movements of the gantry and/or of the couch makes possible the intersection of multiple successive radiation beams at the target location (at the so-called isocentre), thus producing a high total dose inside the target and at the same time, resulting in lower radiation in the surrounding areas.

Some other systems, namely the Cyberknife, commercialized by the company Accuray, uses a small-size LINAC mounted on a robotic arm, allowing large freedom in the motion of the robot head holding the LINAC, and thus allowing a large variety of the LINAC locations and incidence angles.

There exist also IR machines, for example Elekta's VMAT (Volumetric Modulated Arc Therapy) systems, in which the radiation dose is not delivered by a discrete number of incidences, but the radiation beam source sweeps in continuous arcs around the patient's body, while the beams parameters are controlled at the same time. These systems attempt to reduce the treatment times.

It is known to use a number of imaging or positioning techniques to control in real time the radiation delivery, also in consideration of the movements of the patient and/or of the target organ. It is for example known to monitor the position of the target region, for example by fluoroscopy, and to gate the radiation source with a signal that confirms that the target is in a nominal position. These systems can for example suspend the irradiation if the patient or the target organ moves more than a predetermined threshold. Yet, the very large number of input variables and the difficulties of controlling the radiation beam in real time have so far limited the effectiveness of these efforts, and selective treatment of localized areas of the body remains a difficult goal that current devices can attain only in part.

In all those systems, a planning phase is necessary to determine, in the most general case, the number, location, incidence angle, shape and weight(s) of the successive irradiation shots in order to deliver the desired dose profile to the target region while, if necessary, protecting surrounding sensitive regions from a too high irradiation dose.

In the context of the present invention, a shot (or dose shot) is then a radiation dose delivered from a given location, incidence angle, with a given shape and weight(s). A treatment session can comprise a plurality of shots of different sizes and shapes. By extension, a 'shot' could also correspond to an arc in a Modulated Arc therapy.

Depending on the type of system, the parameters to be defined may be more restricted than those described above. As an example, when the LINAC is mounted on a rotating gantry with a fixed couch, the set of incidence angles is restricted to those produced by the rotation of the gantry.

For each shot, the user, i.e. the doctor(s) and/or the physicist, has to determine its location and incidence angle in the target area, as well as the size and shape of the irradiation dose to be delivered around the isocentre.

For each shot, the time of irradiation in relation of the dose-rate of the sources (i.e. the time during which the LINAC is working) must be determined. In the most advanced current systems, such as VMAT (Elekta), and RapidArc (Varian), the dose rate (i.e. the amount of radiation per unit of time) and other parameters of the shot, e.g., in a non-limiting way, the profile of the irradiation in the dose's area (e.g. a Gaussian profile, a flat profile, etc.) may also require prior determination. In the planning phase, each patient's treatment plan is generally developed by a radio-oncologist working in conjunction with a physicist. According to the most widely used planning procedure, they determine, through an iterative process of trial and error, the number, location and incidence angle of shots, along with their size, shape, and weight, and most recently dose rate.

Known radiation inverse treatment planning systems for LINACs and other RT equipment require, in general, that the number, location and incidence angle of shots be given as input parameters, and compute an optimal combination of irradiation parameters respecting a desired dose distribution. As already mentioned, some planning and control system take into account, to some extent, of the movements of the patient when delivering the radiation, e.g. as the patient breathes, or of one or more moving organs of the patient. However the know systems are not able to perform a real-time calculation of the shots, to adapt them to these movements with sufficient precision and often limit themselves to inhibiting shots that would otherwise be off-target due to the patient's movements.

Known radiation inverse treatment planning systems are not sufficiently precise, so that the protection of the areas surrounding the target, e.g. a tumour, is not very effective, especially in larger tumours. This requires a plurality of sessions of the radiation treatment, e.g. radiotherapy.

Moreover, the current procedure for the planning step is relatively complex, tedious, unintuitive and slow. The duration of the planning procedure decreases the productivity and increases the cost of every treatment. Its quality depends essentially on the experience of the user. Acquiring this experience requires a long training period.

Indeed, the current way to do the planning requires the definition of technical and operational parameters of the machine that will ultimately produce the desired dose distribution. The relationship between those parameters and the actual dose distribution is not always intuitive. The medical user is thus asked to acquire and exploit a technical expertise, and in most of the cases needs to be help by a medical physicist, while he/she should rather concentrate on the medical aspects of the treatment.

Inverse planning systems attempt to reduce the above burdens by determining the shot weights based on the knowledge of the target region properties (e.g. from CT or MRT images), the operator prescribes a certain dose distribution within the target region and/or certain dose constraints. An automatic inverse planning system finds a set of parameters resulting in a treatment planning which is as close as possible to the predetermined dose distribution.

The classic inverse planning procedure requires then the definition, by the operator, of the target area and the minimum dose that should be delivered to it. Secondarily, the planning system also helps to minimize the dose to the areas to be protected.

The inverse planning is then typically defined as an optimization problem where the technical parameters are automatically searched to minimize a cost function measuring the difference between the desired dose distribution and that actually achieved. Various optimization techniques may be used.

Such inverse planning systems as are used today are time consuming, as they use slow optimisation techniques and require, most of the time, some parts of manual definition of some parameters by a physicist. The process has then to be repeated if the medical oncologist considers the result not satisfactory, requiring more work and time for the physicist team. To further lengthen the process, Inverse planning system rely on simplified models of radiation interaction (the so-called 'pencil-beam' approximation) in order to deliver a result in a manageable time and, once they have converged to a solution this needs to be validated by more precise, but also slower simulations, like for example Monte Carlo simulations.

The known planning system are also in general static, in the sense that many irradiation parameters must be defined in advance. Often, for example, the number of shots and their angles be defined in advance as input parameters. Often, known planning systems have very limited capabilities of adapting to actual delivered doses in the course of the treatment, which reduces their precision.

Among known systems, US2011085643 describes a Radiation therapy inverse treatment planning using a regularization of sparse segments. This document proposes a method to minimize the number of radiation segments (or sub-fields) of a radiation treatment plan. Importantly, however, the authors do not suggest changing or optimizing in any way the number and the parameters of the irradiation fields used in the therapy. Otherwise said, the method presented in this documents invariably produces treatment plans having a predetermined set of fields, for example, (paragraph [0079]) five fields corresponding to gantry angles of 0°, 70°, 145°, 215°, 290°. While the authors propose an algorithm that aims at reducing the number of radiation segments for each one of these fields, the number and incidence angles of the resulting treatment plan are rigidly predetermined.

It is then an aim of the present invention to obviate or mitigate one or more of the aforementioned disadvantages.

Another limitation of the method described by US2011085643 is that it requires at all costs a multi-leaf collimator. The present invention proposes a method that, while especially effective in combination with multi-leaf collimators, can also be usefully applied to RT equipment that does not include this facility. Furthermore, the method of US2011085643 must, to achieve convergence, limit the parameter space to the interior of a Pareto region and, due to this reason, may fail to find good solutions that lie outside this limit.

It is an aim of the present invention to provide a radiation inverse treatment planning system, which can simplify the planning phase of a treatment.

It is an aim of the present invention to provide a radiation inverse treatment planning system able to perform a real-time calculation of the shots, to adapt them to a dynamic target, taking patient's movements and anatomical modifications of the target into account.

It is a further aim of the present invention to provide a radiation treatment planning system that is capable of dynamically adapting the irradiation parameters, comprising for example the number of shots, their angles, and weights, and correct in real time deviations from the planned irradiation pattern.

It is an aim of the present invention to provide a radiation inverse treatment planning system more precise than the known system.

It is an aim of the present invention to provide a radiation inverse treatment planning system, which is an alternative to the existing systems.

The treatment planning system of the present invention is not limited to the use of a linear accelerator (LINAC) as a radiation source, and can use any other type of radiation source, e.g. and in a non-limiting way cobalt sources or proton beams.

In a more general sense, the scope of the present invention is not even limited to the planning of external beam radiation treatments, but includes the planning of other medical procedures to which the inventive method is applicable.

By way of analogy, the method of the invention could be applied to the planning of a treatment of brachytherapy, by considering that the treatment must consists in placing individual radiation sources having optimized locations and intensities. According to one aspect of the invention, the position and intensities of the individual sources could be computed by pre-computing a set of individual sourced, associating to each source a weight, which could be related to its radioactive intensity, and finding the sparsest subset of individual sources that satisfies the constraints determined by the radio-oncologist, for example on the integral dose delivered to the tumour, respectively to the risk organs in proximity.

Also by analogy, the method of the invention could be applied to a procedure of robotic surgery, decomposed in a combination of individual actions of a scalpel, a thermoablation probe, a cryotherapy probe, or any other surgical instrument, by a robotic apparatus. The inventive method could be used, in this case, to determine the sparsest set of actions that fulfils a given number of constraints.

Brief summary of the invention

According to the invention, these aims are achieved by the object of the appended claims, namely by a radiation inverse treatment planning system, comprising:

- a radiation source, configured for delivering individual dose shots, each individual dose shot having a predetermined location and incidence angle inside and/or outside a target area, a size and a shape
- at least a data bus system,
- a memory coupled to the data bus system, wherein the memory comprises a computer usable program code, and
- a processing unit coupled to the data bus system, wherein the processing unit is configured to execute the computer usable program code to

    - pre-compute a set of individual dose shots,
    - associate a weight to each individual dose shot, based on one or several constraints.

In one embodiment, the weight associate to each individual dose shot comprises the time of irradiation.

In another embodiment, the weight associate to each individual dose shot comprises the dose rate.

In another embodiment, the weight associate to each individual dose shot comprises the dose profile.

In another embodiment, the weight associate to each individual dose shot comprises any other parameter of the dose shot.

The radiation inverse treatment planning system of the present invention can use a linear accelerator (LINAC) as a radiation source.

Compared to known methods that optimize the fluence of each individual dose field, i.e. of each dose shot, the present invention proposes a planning method that permits the optimization of the individual shots. In other terms, starting from a set of possible dose shots, characterized by location and incidence angles, as well as size and shape, the method of the invention operates a selection of an optimal subset of individual shots (or fields). It is worth noting that the starting set of possible dose shots can be a very large one, in theory up to including all the possible configuration of the machine. A strong advantage of the method of the invention lies precisely in its ability to extract an optimal plan with a limited number of shots from such a large set of possibilities.

Importantly, the method of the invention can also be used in earlier-generation machines in which the fluence of individual dose fields cannot be modified.

The present invention proposes an automated method for inverse planning radiation treatment system, where the complete dose distribution delivered is modelled as a sparse linear combination of beams chosen from a pre-defined dictionary. Advantageously the one or more constraints can be related to the corresponding resulting dose distribution.

Advantageously the weight may be representative of the time of irradiation of the single or individual dose shot.

The use of a sparsity criterion allows to eliminate many solutions a priori not possible, and then to quickly converge to a solution. The sparsity allows then computations in real-time, so that it is possible to perform a real-time calculation of the shots, to allow interactive planning and to adapt them to the movements of the patient and/or of an organ of the patient, and/or to the relative movement between the physical support of the radiation source (the gantry) and the physical support of the patient (the couch).

Moreover, the system according to the invention is more precise than the known system, allowing to define constraints that are more intuitive and to realize them, so that the protection of the areas surrounding the target, e.g. a tumour, is more effective. This may require few sessions of the radiation treatment, e.g. one to five sessions in radiosurgery, or a larger number of sessions in fractionated radiotherapy when indicated.

In a preferred embodiment, the system comprises

- a first physical support for this radiation source, e.g. the gantry
- a second physical support arranged for receiving a patient, e.g. the couch. The first physical support and the second physical support are arranged to be moved one relative to the other. Advantageously the processing unit executes the computer usable program code to

- find the sparsest subset of individual dose shots, to satisfy these one or more constraints each time that the first physical support is moved relatively to the second physical support and/or each time that the second physical support is moved relatively to the first physical support.

In another embodiment, the processing unit executes the computer usable program code to find the sparsest subset of individual dose shots, to satisfy this one or more constraints each time that a patient and/or an organ and/or a part of an organ of the patient moves and/or changes its shape and/or size.

In another embodiment, the processing unit executes the computer usable program code to find the sparsest subset of individual dose shots, to satisfy this one or more constraints each time that a patient and/or an organ and/or a part of an organ of the patient moves, alters its shape, or changes its size.

In another embodiment, the processing unit executes the computer usable program code to find the sparsest subset of individual dose shots, to satisfy these one or more constraints each time that the real dose distribution in the patient at a given moment is different from the planned dose distribution.

In a preferred embodiment, the constraint comprises at least the coverage of the whole of part of the target region by a desired dose distribution. Further constraints may be added to modify the dose distribution outside of the target volume, and to limit the maximal dose to defined structures. Constraints may also be added to define the dose distribution within the target volume, if desired.

According to one aspect of the invention, the processing unit executes the computer usable program code to find the sparsest subset of shots to satisfy the constraint(s).

The inventive system according to the invention allows a drastic simplification of the radio-surgical planning via real-time inverse planning system.

In a preferred embodiment, the processing unit executes the computer usable program code to find the minimum number of non-zero weights to satisfy said one or more constraints.

The inventive system according to the invention allows calculating the optimal technical parameters of irradiation to achieve the constraints imposed on the dose distribution. Considering the number of parameters that can be defined by the user during a manual planning, the optimal solution is in practice almost impossible to find, especially in the treatment of complex shape targets, even by an experienced user.

The inventive system according to the invention allows the user to define interactively the constraints on the dose to be delivered, in coverage, magnitude and gradients at the edges of the target or anywhere else in the volume of interest.

The advantages for the user are at least the following:

- He/she does not have to concentrate on the technical aspect of the realization of the desired dose distribution, but only has to consider which dose he/she wants to administer, and where.
- The interactive planning tool allows him/her to decide and modify in real-time the shape of the dose distribution to ensure proper irradiation of the target and proper protection of other organs.
- The planning becomes intuitive, fast, and thus cost-effective.
- The user can also easily add more constraints on the problem, such as a maximal treatment duration, the system providing the best possible planning to be as close as possible to the desired dose distribution while remaining within the time budget, for example.

The planning procedure performed by the system according to the invention is much simpler, faster and more user friendly than the known solutions, especially in complex target configurations.

A convex constrained optimization problem can be used to determine the treatment plan, i.e. the number of beams as well as the beam orientations, sizes, shapes and weights (or a subset of those parameters, depending on the physical properties of the considered system), to produce a desired dose delivery profile.

The optimization problem can include dose constraints applied to both the target region and other areas such as sensitive structures to be protected against high radiation dose.

A dictionary can be computed, composed by a large set of beams covering totally or partially the set of possible beam locations, incidence angles, sizes and shapes. After this computation, a convex optimization problem can be solved to determine the optimal plan, i.e. the optimal subset of those beams as well as their amplitude, to meet the defined constraints.

The present invention concerns also a computer program product, comprising:

a tangible computer usable medium including computer usable program code for a radiation inverse treatment planning system comprising a radiation source configured for delivering individual dose shots, each individual dose shot having a predetermined location and incidence angle inside and/or outside a target area, a size and a shape, the computer usable program code being used for

- pre-compute a set of individual dose shots,
- associate a weight to each individual dose shot, based on one or more constraints, e.g. on the corresponding resulting dose distribution, characterised in that the processing unit executes the computer usable program code to
- find the sparsest subset of individual dose shots, to satisfy said one or more constraints.

The present invention concerns also a computer data carrier storing presentation content created with a radiation inverse treatment planning method, comprising the following steps:

- pre-compute a set of individual dose shots, each individual dose shot being generated by a radiation source, and having a predetermined location and incidence angle inside and/or outside a target area, a size and a shape,
- associate a weight to each individual dose shot, based on one or several constraints, e.g. on the corresponding resulting dose distribution, characterised in that the processing unit executes the computer usable program code to
- find the sparsest subset of individual dose shots, to satisfy said one or more constraints.

Brief Description of the Drawings

The invention will be better understood with the aid of the description of an embodiment given by way of example and illustrated by the figures, in which:

- Fig. 1 is the illustration of an embodiment of a data processing system in which the computer usable program code of the computer program product in accordance with an embodiment of the present invention can be implemented.

- Fig. 2 shows a flow-chart representation of a method which can be implemented in an embodiment of the radiation inverse treatment planning system according to the present invention.

- Figure 3 is a transverse tomographic image of a human pelvis for a simulated prostate cancer patient, showing contours of target organs and organs at risk.

- Figure 4 is a 3D model of the target organs and of the organs at risk.

- Figures 5a to 5c illustrate dose distributions for a 5-beam treatment on the same simulated patient, in transverse, respectively sagittal and coronal section.

- Figure 6 is a dose distribution histogram for a conventional 5-beam treatment and one obtained by the method of the present invention.

- Figures 7a to 7c illustrate dose distributions for a 10-beam treatment on the same simulated patient, in transverse, respectively sagittal and coronal section obtained by the inventive method.

- Figure 8 is a comparison, on a dose distribution histogram, between a 5-beam treatment and a 10-beam treatment.

Detailed Description of possible embodiments of the Invention

Although the present invention will be described in more detail in connection with a LINAC as radiation source, the present invention finds applicability of connection with many other sources, as explained here above. For example, it can use other radiation sources, as gamma-ray sources, for example $^{60}$Co sources, X-ray or brehmsstrahlung radiations in the keV or MeV regions or beams of particles, protons in particular, but also ions, electrons, or any other suitable radiation capable of delivering a radiation dose to a target

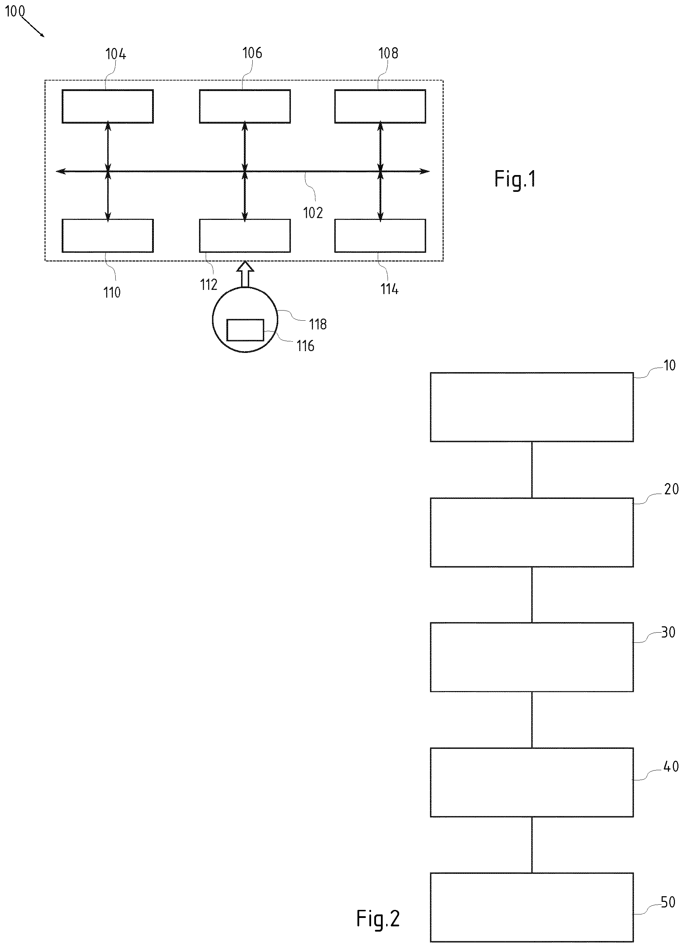

Fig. 1 is the illustration of an embodiment of a data processing system 100 in which the computer usable program code of the computer program product in accordance with an embodiment of the present invention may be implemented.

The radiation inverse treatment planning system 100 according to the invention comprises:

- a radiation source (not visible),
- at least a data bus system 102,
- a memory 106 coupled to the data bus system 102, wherein the memory comprises a computer usable program code, and
- a processing unit 104 coupled to the data bus system 102.

Fig. 2 shows a flow-chart representation of a method which can be implemented in an embodiment of the inverse treatment planning system 100 according to the present invention.
Advantageously, the processing unit 102 is configured to execute the computer usable program code to

- pre-compute a dictionary composed of a list (or set) of possible dose shots' locations, incidence angles, sizes and shapes (step 10),
- define by the user the desired dose in the target area and potential additional constraints, for instance on the areas to be protected from too high dose radiation (step 20),
- solve a convex problem to determine the plan, i.e. to find which of those shots, and with which weights, will be actually used (steps 30, 40 and 50).

In one preferred embodiment, the set of precomputed dose shots (step 10) can be located on a discrete three-dimensional (3D) grid of fixed resolution in a 3D space.

As discussed, the first step of figure 1 (step 10) is to build a list dictionary of possible dose shots (or dose distributions patterns) located (centered) at all possible locations and incidence angles on a 3D grid, or a subset of them (e.g. those located only within the target area).

In one preferred embodiment, two consecutive locations on this grid in each of the three dimensions are spaced by a distance less than 1 mm, e.g. 0.5 mm.

The dictionary is thus the set of functions

$$\left\{ a^{j} \right\}_{j=1}^{N}$$

with N denoting the size of the dictionary.

Each component $a^j$ of the dictionary will be named "atom".

The complete dose distribution can be calculated as the weighted sum of the contributions from each atom. The dose $d$ at any point $(x, y, z)$ of the 3D space can be computed as

$$d(x, y, z) = \sum_{j=1}^{N} s_j a^j (x, y, z)$$

where $s_j$ denotes the weight associated to the j-th atom.

For example, for a given system using a rotating gantry and a moving couch, the dictionary can be obtained by discretizing the rotation angles of the gantry and the positions of the coach to create a discrete grid on the sphere and considering different beam sizes and shapes for each discrete location and orientation.

As another example, for a given LINAC location and orientation, the beam going through a multi-leave collimator can be discretized as a series of small discrete "beamlets", parallel to each other, each of them with their own weight that has to be determined. For specific newer systems, dose rate modulation can also be discretized.

In one preferred embodiment, this step can be performed by considering pre-calculated individual dose profiles, produced by a set of individual beams with different locations, orientations, sizes and shapes, and by translating them to all the considered grid points. This step can also be performed by taking into account the physical properties of the patient's anatomy, based for instance on the medical images acquired for the planning.

The objective of the inverse planning method is to find the minimum number of non-zero weights $s_j$ so that the constraints imposed by the user at step 20 are satisfied.

The complete dose distribution $d$ can be calculated at a predefined number of points in the 3D space, for instance on a pre-defined grid $G$ of $P$ points.

This dose distribution $d$ can be represented by a vector f of dimension $P$ that can be defined as

$$f = As$$

where A is an $P \times N$ matrix whose columns are the value of the dose delivered by each atom at each point of the grid $G$, and s is a vector of the weights of the atoms, of dimension $N$.

According to the invention, s has to be sparse, i.e. the number $K$ of non-zero coefficients of s has to be much smaller than $N$. In a typical example, N may be as big as 100'000 or more, while K may be as small as 50 or less.

The positions of the non-zero elements in s determine which atoms in the dictionary will be used in the treatment, i.e. they determine the actual shot shapes and their locations.

The values of s determine the shot weights.

Once building the dictionary $A$ (step 10 in Fig. 2), a vector s with minimum number of non-zero elements is computed by satisfying the dose constraints defined by the user in step 20.

It must be understood that, even if the dose constraints in Fig. 2 are inputted by the user after the pre-computation of the dictionary, this inputting can be performed before the pre-computing step 10.

A possible optimization criterion is finding a plan that minimizes a weighted L1 norm of vector s (i.e. the sum of the elements of the vector s) and meets all the dose constraints. The weighted L1 norm of s is closely related to the treatment time. This optimization problem can advantageously be formulated as a convex optimization problem (step 50), as only the weights of the individual dose shots are optimized (in fact simultaneously optimize the locations, sizes, shapes, and weights of the individual dose shots to guarantee a dose constraint will result in a non-convex optimization problem). In another embodiment, it is find a plan that minimizes a weighted L0 norm of vector s (i.e. the number of the elements of the vector s that are different from zero) and meets all the dose constraints. In another embodiment, the optimization criterion may be finding a plan that minimizes a weighted L2 norm of vector s and meets all the dose constraints.

Let $T$ denote the set of indexes of the vector $f$ corresponding to points that belong to the target region, let $R$ denote those belonging to the sensitive areas to be protected, and $Q$ the set of remaining indexes. In addition, letting $a_i$ denotes the i-th row of the matrix $A$. The i-th component of the vector $f$ can be expressed as

$$f_i = a_i s$$

i.e. the inner product of the i-th row of the dictionary $A$ and the vector s. Thus, the optimal plan is computed by solving the following convex problem:

$$\min \|s\|_{1,w} \text{ such that } \begin{cases} a_i s \geq b_{\min} & \forall i \in T \\ a_i s \leq b_{\max} & \forall i \in R \\ a_i s \leq b_{\min} & \forall i \in Q \\ s \geq 0 \end{cases}$$

where

$$\|s\|_{1,w} = \sum_{i=1}^{N} w_i |s_i|$$

denotes the weighted L1 norm of the vector s with weights $w_i \geq 0$, $b_{min}$ is the minimum dose at the target region $T$, $b_{max}$ is the maximum allowed dose at sensitive regions $R$, and $s \geq 0$ denotes the positivity constraint on the values of s.

Additional constraints can be added at step 20 to the formulation as equality or inequality constraints. This can for instance be related to a desired dose gradient index, or to different values of the minimal dose delivered to different parts of the target region, or to different values of the maximal dose delivered to regions to be protected. This optimization problem can then be solved by any convex optimization method, for instance by convex linear programming algorithms.

The weighted L1 norm is a convex function that promotes sparse solutions, i.e. solving this constrained minimization problem will determine the sparsest vector s that meets all the dose constraints.

Minimizing the number of beams and the sum of their weights is akin to minimize the treatment time. Other types of convex penalties that promote structured sparsity, such as the L0, L1 or L2 norm that promotes group sparsity, can be employed. The idea behind this approach is to leverage from the particular structure of a particular LINAC technique.

This optimization problem can then be solved by any convex optimization method, for instance by convex linear programming algorithms.

The inventive system proposes then an inverse treatment planning system wherein the complete dose distribution is modeled as a sparse linear combination of single shot dose chosen from a pre-computed dictionary or library of pre-computed single shot doses.

A convex constrained optimization procedure is used to determine the treatment plan. The shot weights are optimized, under sparsity constraint, to guarantee that the constraints on the dose distribution be met.

The optimization procedure does not require the user to provide initial shot locations, and the convex optimization formulation can include dose constraints applied both to the target region and to other areas such as sensitive structures to be protected against too high radiation doses.

Importantly, the optimization procedure of the invention is computationally advantageous with respect to other planning methods hitherto employed. It becomes therefore possible to repeat the optimization during the treatment, in real time, to take anatomical variations, movements, or deviations from the predetermined plan into account.

According to a variant, the radiation planning system of the invention is equipped or is connectable with imagery or position sensors that allow capturing the irradiation target during the treatment and that provide an information when the geometry of the target region changes. The system is adapted to recognize when the shape, position, and dimension of the target organ, or of a part of an organ, change beyond predetermined thresholds, for example due to patient involuntary movements, breathing, or due to of any other reason. The radiation inverse treatment planning system of the invention is arranged to repeat the optimization procedure as delineated above, starting from the previously chosen solution, and find a new solution for the updated target configuration.

Furthermore, the system can: determine the real delivered dose distribution based on the sensor's information and/or on information and signals provided by appropriate dosimetry units; compare the delivered dose with what was predetermined in the treatment plan; adapt the constraint of the optimization algorithm in function of this comparison; and repeat or refine the optimization in order to achieve more precisely the desired dose distribution.

Fig. 1 is an embodiment of a system 100 according to the invention. The system 100 of Fig. 1 may be located and/or otherwise operate at any node of a computer network, that may exemplarily comprise clients, servers, etc., and it is not illustrated in the figure. In the embodiment illustrated in Fig. 1, the system 100 includes communications fabric 102, which provides communications between processor unit 104, memory 106, persistent storage 108, communications unit 110, input/output (I/O) unit 112, and display 114.

Processor unit 104 serves to execute instructions for software that may be loaded into memory 106. Processor unit 104 may be a set of one or more processors or may be a multi-processor core, depending on the particular implementation. Further, processor unit 104 may be implemented using one or more heterogeneous processor systems in which a main processor is present with secondary processors on a single chip. As another illustrative example, the processor unit 104 may be a symmetric multi-processor system containing multiple processors of the same type.

In some embodiments, the memory 106 shown in Fig. 1 may be a random access memory or any other suitable volatile or non-volatile storage device. The persistent storage 108 may take various forms depending on the particular implementation. For example, the persistent storage 108 may contain one or more components or devices. The persistent storage 108 may be a hard drive, a flash memory, a rewritable optical disk, a rewritable magnetic tape, or some combination of the above. The media used by the persistent storage 108 also may be removable such as, but not limited to, a removable hard drive.

The communications unit 110 shown in Fig. 1 provides for communications with other data processing systems or devices. In these examples, communications unit 110 is a network interface card. Modems, cable modem and Ethernet cards are just a few of the currently available types of network interface adapters. Communications unit 110 may provide communications using either or both physical and wireless communications links.

The input/output unit 112 shown in Fig. 1 enables input and output of data with other devices that may be connected to the system 100. In some embodiments, input/output unit 112 may provide a connection for user input through a keyboard and mouse. Further, input/output unit 112 may send output to a printer. Display 114 provides a mechanism to display information to a user.

Instructions for the operating system and applications or programs are located on the persistent storage 108. These instructions may be loaded into the memory 106 for execution by processor unit 104. The processes of the different embodiments may be performed by processor unit 104 using computer implemented instructions, which may be located in a memory, such as memory 106. These instructions are referred to as program code, computer usable program code, or computer readable program code that may be read and executed by a processor in processor unit 104. The program code in the different embodiments may be embodied on different physical or tangible computer readable media, such as memory 106 or persistent storage 108.

Program code 116 is located in a functional form on the computer readable media 118 that is selectively removable and may be loaded onto or transferred to the system 100 for execution by processor unit 104. Program code 116 and computer readable media 118 form a computer program product 120 in these examples. In one example, the computer readable media 118 may be in a tangible form, such as, for example, an optical or magnetic disc that is inserted or placed into a drive or other device that is part of persistent storage 108 for transfer onto a storage device, such as a hard drive that is part of persistent storage 108. In a tangible form, the computer readable media 118 also may take the form of a persistent storage, such as a hard drive, a thumb drive, or a flash memory that is connected to the system 100. The tangible form of computer readable media 118 is also referred to as computer recordable storage media. In some instances, computer readable media 118 may not be removable.

Alternatively, the program code 116 may be transferred to the system 100 from computer readable media 118 through a communications link to communications unit 110 and/or through a connection to input/output unit 112. The communications link and/or the connection may be physical or wireless in the illustrative examples. The computer readable media also may take the form of non-tangible media, such as communications links or wireless transmissions containing the program code.

The different components illustrated for data processing system 100 are not meant to provide architectural limitations to the manner in which different embodiments may be implemented. The different illustrative embodiments may be implemented in a data processing system including components in addition to or in place of those illustrated for data processing system 100. Other components shown in Fig. 1 can be varied from the illustrative examples shown. For example, a storage device in the system 100 is any hardware apparatus that may store data. Memory 106, persistent storage 108, and computer readable media 118 are examples of storage devices in a tangible form. Example of determination of a treatment plan

Classically, RT planning can be decomposed as follows :

a. An oncologist establishes a diagnosis of cancer, and chooses a suitable form of therapy, in particular a radiation therapy. The present example assumes that the diagnosis is of a prostate cancer with an indication for external beam radiotherapy.

b. The patient undergoes a CT scan, or another medical imaging procedure. Tomographic images of the target volume are forwarded to the planning system.

c. A radiation oncologist determines the target dose that needs to be delivered to the tumour, sets upper limits to the dose that can be deposited in the neighbouring risk organs and tissues, and the number of fractions needed to realize the treatment.

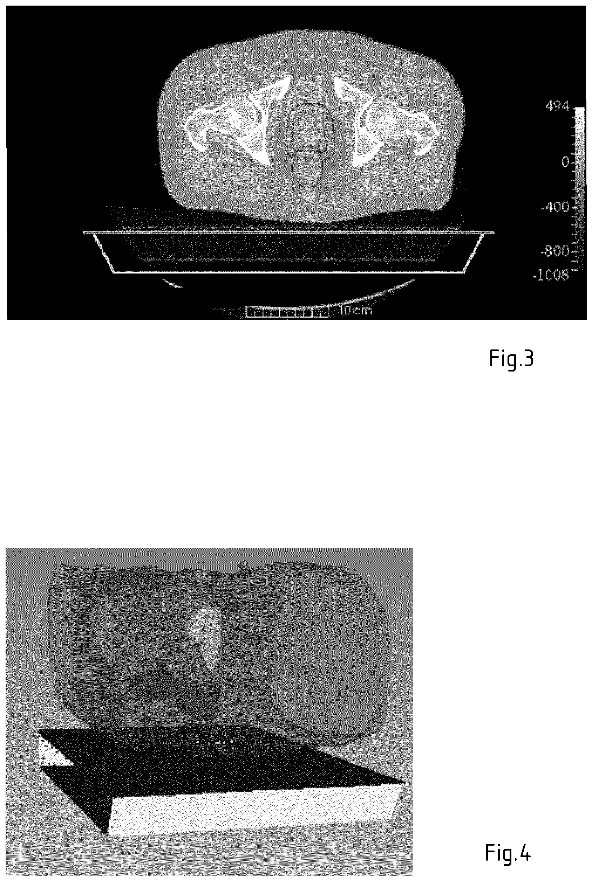

d. A radio-physicist, or a radio-oncologist delineates the contours of the target and of the neighbouring organs at risk. In most cases, he also traces a contour defining a guard volume outside, but immediately adjacent to the target. Figure 3 illustrates a transverse section of the pelvis showing the contours determined for the prostate 201, the guard volume 205, and the organs at risk: rectum 215, and bladder 225. Figure 4 is a 3D reconstruction of the contours based on a plurality of tomographic slices. This task may be carried out by using several specialised pieces software and contouring anatomic atlases

e. .

f. Based on the clinical target and limit doses determined in step c, a radio-physicist determines the boundary conditions for the optimization problem.

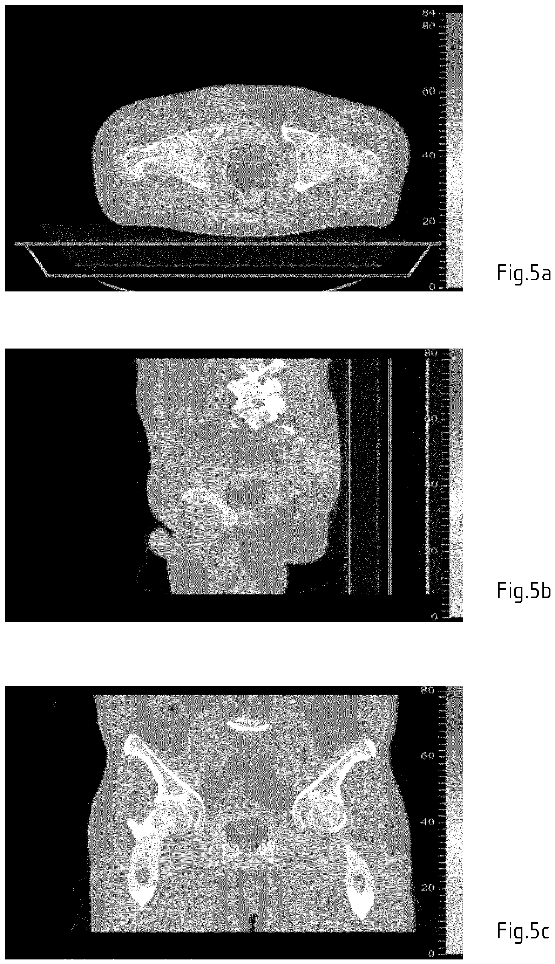

g. Usually, the radio physicist determines at this stage the number and the orientation of the radiation beams or, in the case of a VMAT therapy, of the radiation arcs. This choice is often based on experience and practice and is therefore operator-dependent. In any case, the operator will limit his effort to a reduced solution space that he or she can manage. Having determined the number of beams and the boundary conditions, the operator applies an inverse planning program to determine the fluence maps of each beam and the parameters of aperture of the multi-blade collimators. At this stage, the algorithm are based on simplified models of the radiation interaction, typically a pencil-beam algorithm, to keep the execution time within acceptable limits but, even so, the computing times are in general of some minutes, or dozens of minutes for one plan simulation. In some cases, faster processing times can be obtained starting from a previous solution without changing the number and angles of the beams. Still this phase is exceedingly time-consuming. The dose distributions so obtained are shown in figures 5a-5c

h. Once the radio-physicist obtains a plan that fits with the clinical targets, he often runs a Monte Carlo simulation to improve the precision of the dose distribution and validate the plan.

i. A sequencer software translates the fluence maps into programs for the positioning of the blades in the multi-blade collimators. This needs to take into account several parameters like the number and thickness of the blades, their maximal translation speed, and their transmission coefficients and so on.

j. The plan may be further checked in a quality control step, which involves measuring the effective doses in a water phantom.

k. The effective treatment may then take place

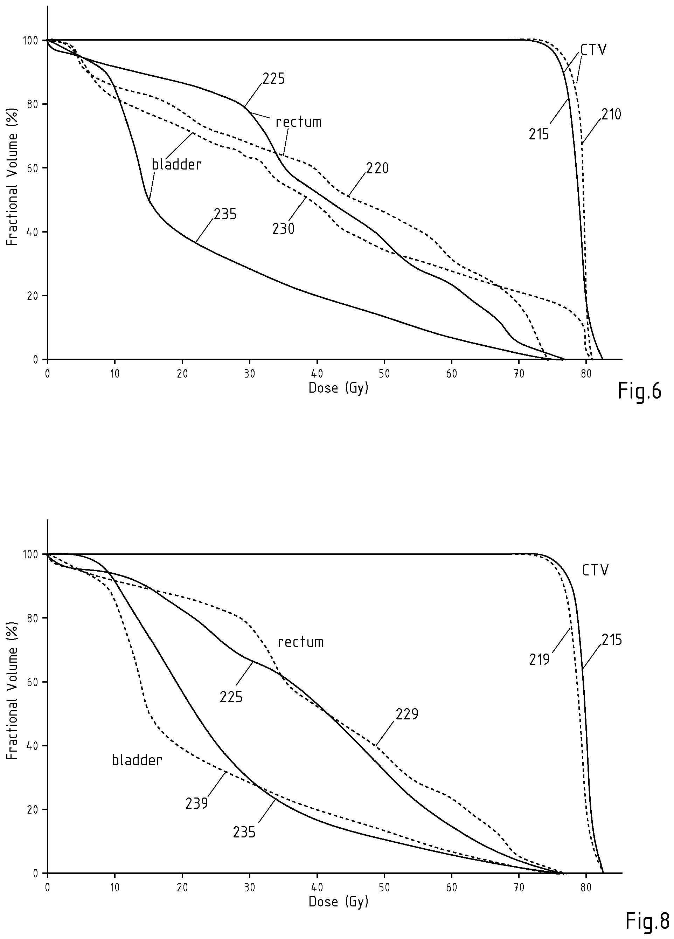

The method of the invention allow automatizing the step of determining the number of beams-arcs and their orientation of step f. above. It allows also computing an optimal solution among a great number of solutions much faster than the methods used in the art. The operator can obtain the best plan in a matter of seconds rather than in minutes or dozen of minutes. Also, the method of the invention can find optimal plans with a work with a large number of beams: a task very difficult to tackle by human operators. Figure 6 illustrates a comparison between a treatment plan determined by an expert radio physicist (dashed lines) and one found by the method of the invention. Both plans have five beams. A comparison shows that the human-designed plan and the one obtained by the invention allow achieving the target dose in the target (curves 210 and 215). The analysis of the organs at risk shows that the rectum (curves 220 for the human-computed plan, respectively 225 for the plan derived by the method of the invention) receives in both cases an equivalent dose, while the bladder (curves 230 and 235) is much better preserved by the plan issued by the inventive method. As the plan is obtained in a few seconds, the radio physicist has the possibility of exploring a larger space of solutions and test variants, like changing the maximum number of beams, the contours, or the boundary conditions.

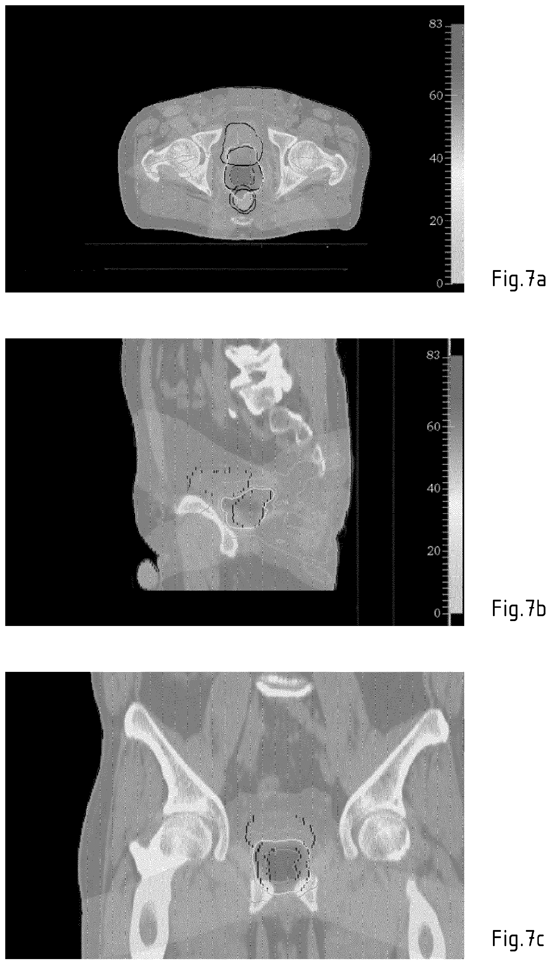

The algorithm can also devise plans with more beams that could hardly be managed by conventional means. Figures 7a-7c, for example, show dose distributions for a 10-beams plan obtained by the method of the invention, in the same simulated patient. This leads to a better plan, especially for what the doses in the organs at risk are concerned. Figure 8 shows a comparison between a ten-beam plan (dashed), indicated by distribution curves 219, 229, and 239 and a five-beam plan (solid; curves 215, 225, 235). It can be appreciated that the ten-beam plan is superior both in term of the irradiation of the target and of the protection of the organs at risk.

Importantly, the number of beams and their orientation can be left free in the algorithm of the invention. It is however possible to set an upper bound to the number of beams, since treatments with many beams have more movement phases and tend to be longer. If the length of the treatment is not a concern, the method of the invention can be let run with an unconstrained number of beams and will provide the plan that meets the targets and boundary conditions using a minimal number of beams, thanks to the choice of a maximally sparse solution.

According to an embodiment, the system according to the invention is implemented on a processing unit (CPU) of a single computer. In another embodiment, it is implemented on a multi-cores computer, the cores working in parallel. In another embodiment, it is implemented on a Graphic Processing Unit (GPU) of a computer. In another embodiment, it is implemented on a plurality of computers, which work totally or partially in parallel.

According to an independent aspect of the invention, the system according to the invention can be shared in innovative training scenarios (including tele-training and remote coaching). In one embodiment, the interactive inverse planning is provided as a tele-service, the system running in a processing centre accessed by the users over secured Internet connections.

**Reference numbers used in the figures**

| | |
|---|---|
| 10 | Pre-computing step |
| 20 | User inputting step (constraints) |
| 30 | Sparsity step |
| 40 | Association step |
| 50 | Optimization step |

| | |
|---|---|
| 100 | System |
| 102 | Data bus system |
| 104 | Processing unit |
| 106 | Memory |
| 108 | Persistent storage |
| 110 | Communication unit |
| 112 | I/O unit |
| 114 | Display |
| 116 | Program code |
| 118 | Computer readable media |

**Claims**

1. A radiation inverse treatment planning system for a linear accelerator, comprising:

   - a radiation source, configured for delivering individual dose shots ($a^j$), each individual dose shot having a predetermined location and incidence angle inside and/or outside a target area, a size and a shape
   - at least a data bus system (102),
   - a memory (106) coupled to the data bus system (102), wherein the memory (106) comprises a computer usable program code, and
   - a processing unit (104) coupled to the data bus system (102), **characterised in that** the processing unit (104) is configured to execute the computer usable program code to

   - pre-compute (10) a dictionary composed by a set of individual possible dose shots ($a^j$) including, for each dose shot, its location, incidence angle, size and shape,
   - associate (40) a weight ($s_j$) to each individual dose shot ($a^j$), based on one or more dose constraints (20), so as to get individual weighted dose shots,
   - apply a convex optimization criterion (50), and
   - find (30) the sparsest subset of said individual weighted dose shots of non-zero weights to satisfy said one or more dose constraints (20).

2. The system of claim 1, the weight ($s_j$) associated to each individual dose shot ($a^j$) comprising the time of irradiation.

3. The system of claim 1, the weight ($s_j$) associated to each individual dose shot ($a^j$) comprising the dose rate.

4. The system of claim 1, the weight ($s_j$) associated to each individual dose shot ($a^j$) comprising dose profile.

**5.** The system of one of claims 1 to 4, comprising:

- a first physical support for said radiation source
- a second physical support arranged for receiving a patient

wherein the first physical support and the second physical support are arranged to be moved one relative to the other

and wherein the processing unit (104) executes the computer usable program code to

- find (30) the sparsest subset of said individual weighted dose shots of non-zero weights, to satisfy said one or more dose constraints (20) each time that the first physical support is moved relatively to the second physical support and/or each time that the second physical support is moved relatively to the first physical support.

**6.** The system of one of the claims 1 to 5, wherein the processing unit (104) executes the computer usable program code to

- find (30) the sparsest subset of individual dose shots, so as to satisfy said one or more dose constraints (20) each time that a patient and/or an organ and/or a part of an organ of the patient moves, and/or alters its shape and/or changes its dimensions.

**7.** The system of one of the claims 1 to 5, connectable with sensors for capturing the irradiation target during the treatment, detecting changes in the geometry and/or the shape and/or the position and/or the dimensions of the target region, and updating a target configuration accordingly, wherein the processing unit (104) executes the computer usable program code to

- find (30) the sparsest subset of individual dose shots, to satisfy said one or more dose constraints (20) upon said changes, based on said updated target configuration.

**8.** The system of one of the claims 1 to 5, wherein the processing unit (104) executes the computer usable program code to

- find (30) the sparsest subset of individual dose shots, to satisfy said one or more dose constraints (20) each time that the real dose distribution in the patient at a given moment is different from the planned dose distribution.

**9.** The system of one of claims 1 to 7, wherein the number of non-zero weights ($s_j$) is at least 1/100 of the number of pre-computed individual dose shots ($a^j$).

**10.** The system of one of claims 1 to 9, wherein the processing unit (104) executes the computer usable program code to

- minimize a weighted L1 norm of the vector of weights while satisfying said dose constraints (20), to obtain an optimal subset of individual dose shots.

**11.** The system of one of claims 1 to 10, wherein the processing unit (104) executes the computer usable program code to

- minimize a weighted L2 norm of the vector of weights while satisfying said dose constraints (20), to obtain an optimal subset of individual dose shots.

**12.** The system of one of claims 1 to 11, wherein the processing unit (104) executes the computer usable program code to

- locate each individual dose shot ($a^j$) in a location of a three-dimensional grid (G).

**13.** The system of one of claims 1 to 12, wherein the processing unit (104) is configured to execute the computer usable program code in real-time.

**14.** The system of one of claims 1 to 13, wherein said one or more dose constraints (20) comprise dose constraints applied to the target region (T) and/or to other areas such as sensitive structures (R) to be protected against too high dose radiation.

**15.** The system of one of claims 1 to 14, wherein the processing unit (104) is configured to execute the computer usable program code to

- take into account the physical properties of the patient's anatomy during the pre-computing of said dictionary.

**16.** The system of one of claims 1 to 15, said optimization criterion (50) comprising minimizing a treatment time.

**17.** The system of one of claims 1 to 16, wherein the radiation source is a linear accelerator.

**18.** The system of one of claims 1 to 17, wherein the radiation source is a cobalt source or a proton beam.

**19.** A computer program product, comprising:

a tangible computer usable medium including computer usable program code for a radiation inverse treatment planning system comprising

a radiation source configured for delivering individual dose shots (a$^j$), each individual dose shot having a predetermined location and incidence angle inside and/or outside a target area, a size and a shape, **characterised in that** the computer usable program code is used to

- pre-compute (10) a dictionary composed by a set of individual possible dose shots (a$^j$) including, for each dose shot, its location, incidence angle, size and shape,

- associate (40) a weight (s$_j$) to each individual dose shot (a$^j$), based on one or more dose constraints (20), so as to get individual weighted dose shots wherein the processing unit (104) executes the computer usable program code to
-- apply a convex optimization criterion (50), and
- find (30) the sparsest subset of said individual weighted dose shots of non-zero weights to satisfy said one or more dose constraints (20).

**Patentansprüche**

1. Inverses Behandlungsplanungssystem für einen Linearbeschleuniger, umfassend:

- eine Strahlungsquelle, die so konfiguriert ist, um individuelle Dosisaufnahmen (a$^j$) zu liefern, wobei jede individuelle Dosisaufnahme einen vorbestimmten Ort und Einfallswinkel innerhalb und/oder ausserhalb eines Zielbereichs, eine Grösse und eine Form hat,
- mindestens ein Datenbussystem (102),
- einen mit dem Datenbussystem (102) gekoppelten Speicher (106), worin der Speicher (106) einen computerverwendbaren Programmcode umfasst, und
- eine mit dem Datenbussystem (102) gekoppelte Verarbeitungseinheit (104), **dadurch gekennzeichnet, dass** die Verarbeitungseinheit (104) konfiguriert ist, um den computerverwendbaren Programmcode auszuführen, zum

- Vorberechnen (10) eines Wörterbuchs, das sich aus einem Satz von individuellen Dosisaufnahmen (a$^j$) zusammensetzt, einschliesslich für jede Dosisaufnahme: deren Ort, Einfallswinkel, Grösse und Form,
- zu jeder individuellen Dosisaufnahme (a$^j$), Zuweisen (40) eines Gewichts (s$_j$) auf der Basis von einer oder mehreren Dosisbeschränkungen (20), um individuelle gewichtete Dosisaufnahmen zu erhalten,
- Anwenden eines konvexen Optimierungskriteriums (50), und
- Finden (30) der spärlichsten Teilmenge von den besagten individuellen gewichteten Dosisaufnahmen mit Gewichten ungleich Null, um die besagte(n) eine oder mehreren Dosisbeschränkungen (20) zu erfüllen.

2. System gemäss Anspruch 1, wobei das jeder individuellen Dosisaufnahme (a$^j$) zugewiesene Gewicht (s$_j$) die Bestrahlungszeit umfasst.

3. System gemäss Anspruch 1, wobei das jeder individuellen Dosisaufnahme (a$^j$) zugewiesene Gewicht (s$_j$) die Dosisrate umfasst.

4. System gemäss Anspruch 1, wobei das jeder individuellen Dosisaufnahme (a$^j$) zugewiesene Gewicht (s$_j$) das Dosisprofil umfasst.

5. System gemäss einem der Ansprüche 1 bis 4, umfassend:

- eine erste physikalische Unterstützung für die Strahlungsquelle
- eine zweite physikalische Unterstützung, zum Empfangen eines Patienten eingerichtet,

worin die erste physikalische Unterstützung und die zweite physikalische Unterstützung angeordnet sind, um in Bezug auf einander bewegt zu werden, und worin die Verarbeitungseinheit (104) den computerverwendbaren Programmcode ausführt, zum Finden (30) der spärlichsten Teilmenge von den besagten individuellen gewichteten Dosisaufnahmen mit Gewichten ungleich Null, um die besagte(n) eine oder mehreren Dosisbeschränkungen (20) zu erfüllen, jedes Mal wenn die erste physikalische Unterstützung in Bezug auf die zweite physikalische Unterstützung bewegt wird und/oder jedes Mal wenn die zweite physikalische Unterstützung in Bezug auf die erste physikalische Unterstützung bewegt wird.

6. System gemäss einem der Ansprüche 1 bis 5, worin die Verarbeitungseinheit (104) den computerverwendbaren Programmcode ausführt, zum Finden (30) der spärlichsten Teilmenge von individuellen Dosisaufnahmen, um die besagte(n) eine oder mehreren Dosisbeschränkungen (20) zu erfüllen, jedes Mal wenn ein Patient und/oder ein Organ und/oder ein Teil eines Organs des Patienten sich bewegt und/oder seine Form verändert und/oder seine Grösse ändert.

7. System gemäss einem der Ansprüche 1 bis 5, welches mit Sensoren verbindbar ist, um das Bestrahlungsziel während der Behandlung zu erfassen, Änderungen in der Geometrie und/oder Form und/oder Position und/oder Grösse der Zielregion zu erkennen und eine Zielkonfiguration dementsprechend zu aktualisieren, worin die Verarbeitungseinheit (104) den computerverwendbaren Programmcode ausführt, zum Finden (30) der spärlichsten Teilmenge von individuellen Dosisaufnahmen, um die besagte(n) eine oder mehreren Dosisbeschränkungen (20) zu erfüllen, bei diesen besagten Änderungen auf der Basis der besagten aktualisierten Zielkonfiguration.

8. System gemäss einem der Ansprüche 1 bis 5, worin die Verarbeitungseinheit (104) den computerverwendbaren Programmcode ausführt, zum Finden (30) der spärlichsten Teilmenge von individuellen Dosisaufnahmen, um die besagte(n) eine oder mehreren Dosisbeschränkungen (20) zu erfüllen, jedes Mal wenn die reelle Dosisverteilung im Patienten zu einem bestimmten Zeitpunkt sich von der geplanten Dosisverteilung unterscheidet.

9. System gemäss einem der Ansprüche 1 bis 7, worin die Anzahl von Gewichten ungleich null ($s_j$) mindestens 1/100 der Anzahl von vorberechneten individuellen Dosisaufnahmen ($a^j$) ist.

10. System gemäss einem der Ansprüche 1 bis 9, worin die Verarbeitungseinheit (104) den computerverwendbaren Programmcode ausführt, um eine gewichtete L1-Norm des Vektors von Gewichten zu minimieren, während die besagten Dosisbeschränkungen (20) erfüllt werden, um so eine optimale Teilmenge von individuellen Dosisaufnahmen zu erhalten.

11. System gemäss einem der Ansprüche 1 bis 10, worin die Verarbeitungseinheit (104) den computerverwendbaren Programmcode ausführt, um eine gewichtete LO-Norm des Vektors von Gewichten zu minimieren, während die besagten Dosisbeschränkungen (20) erfüllt werden, um so eine optimale Teilmenge von individuellen Dosisaufnahmen zu erhalten.

12. System gemäss einem der Ansprüche 1 bis 11, worin die Verarbeitungseinheit (104) den computerverwendbaren Programmcode ausführt, um jede individuelle Dosisaufnahme ($a^j$) an einem Ort eines dreidimensionalen Gitters (G) zu lokalisieren.

13. System gemäss einem der Ansprüche 1 bis 13, worin die Verarbeitungseinheit (104) konfiguriert ist, um den computerverwendbaren Programmcode in Echtzeit auszuführen.

14. System gemäss einem der Ansprüche 1 bis 13, worin die besagte eine oder die besagten mehreren Dosiseinschränkungen (20) Dosiseinschränkungen umfassen, welche auf die Zielregion (T) und/oder auf andere Bereiche wie empfindliche Strukturen (R) angewendet werden, die vor Strahlung einer zu hohen Dosis geschützt werden sollen.

15. System gemäss einem der Ansprüche 1 bis 14, worin die Verarbeitungseinheit (104) konfiguriert ist, um den computerverwendbaren Programmcode auszuführen, um die physikalischen Eigenschaften der Anatomie des Patienten während der Vorberechnung des besagten Wörterbuchs zu berücksichtigen.

16. System gemäss einem der Ansprüche 1 bis 15, wobei das besagte Optimierungskriterium (50) das Minimieren einer Behandlungszeit umfasst.

17. System gemäss einem der Ansprüche 1 bis 16, worin die Bestrahlungsquelle ein Linearbeschleuniger ist.

18. System gemäss dem vorhergehenden Anspruch, worin die Bestrahlungsquelle eine Kobaltquelle oder ein Protonenstrahl ist.

19. Computerprogrammprodukt, umfassend:

ein greifbares computerverwendbares Medium mit einem computerverwendbaren Programmcode für ein inverses Strahlungsbehandlungsplanungssystem, umfassend eine Strahlungsquelle, die so konfiguriert ist, um individuelle Dosisaufnahmen ($a^j$) zu liefern, wobei jede individuelle Dosisaufnahme einen vorbestimmten Ort und Einfallswinkel innerhalb und/oder ausserhalb eines Zielbereichs, eine Grösse und eine Form hat, **dadurch gekennzeichnet, dass** der computerverwendbare Programmcode verwendet wird zum:

- Vorberechnen (10) eines Wörterbuchs, das sich aus einem Satz von individuellen Dosisaufnahmen ($a^j$) zusammensetzt, einschliesslich für jede Dosisaufnahme: deren Ort, Einfallswinkel, Grösse und Form,
- zu jeder individuellen Dosisaufnahme ($a^j$), Zuweisen (40) eines Gewichts ($s_j$) auf der Basis von einer oder mehreren Dosisbeschränkungen (20), um individuelle gewichtete Dosisaufnahmen zu erhalten, worin die Verarbeitungseinheit (104) den computerverwendbaren Programmcode ausführt, zum
- Anwenden eines konvexen Optimierungskriteriums (50), und
- Finden (30) der spärlichsten Teilmenge von individuellen gewichteten Dosisaufnahmen mit Gewichten ungleich Null, um die besagte(n) eine oder mehreren Dosisbeschränkungen (20) zu erfüllen.

## Revendications

1. Système de planification inverse de traitement de rayonnement pour un accélérateur linéaire, comprenant :

   - une source de rayonnement, configurée pour délivrer des doses individuelles ($a^j$), chaque dose individuelle ayant un emplacement prédéterminé et un angle d'incidence à l'intérieur et/ou à l'extérieur d'une zone cible, une dimension et une forme,
   - au moins un système de bus de données (102),
   - une mémoire (106) couplée au système de bus de données (102), dans laquelle la mémoire (106) comprend un code de programme utilisable par ordinateur, et
   - une unité de traitement (104) couplée au système de bus de données (102), **caractérisée en ce que** l'unité de traitement (104) est configurée pour exécuter le code de programme utilisable par ordinateur afin de

      - pré-calculer (10) un dictionnaire composé d'un ensemble de tirs individuels de doses possibles ($a^j$) comprenant, pour chaque tir individuel de dose, son emplacement, son angle d'incidence, sa dimension et sa forme,
      - associer (40) un poids ($s_j$) à chaque tir individuel de dose ($a^j$), en fonction d'une ou de plusieurs contraintes de dose (20), de manière à obtenir des tirs individuels de doses pondérés,
      - appliquer un critère d'optimisation convexe (50), et
      - trouver (30) le sous-ensemble le plus creux de tirs individuels de doses pondérés de poids non nuls pour satisfaire à une ou plusieurs contraintes de dose (20).

2. Système selon la revendication 1, le poids ($s_j$) associé à chaque tir individuel de dose ($a^j$) comprenant le temps d'irradiation.

3. Système selon la revendication 1, le poids ($s_j$) associé à chaque tir individuel de dose ($a^j$) comprenant le débit de dose.

4. Système selon la revendication 1, le poids ($s_j$) associé à chaque tir individuel de dose ($a^j$) comprenant un profil de dose.

5. Système selon l'une des revendications 1 à 4 comprenant :

   - un premier support physique pour ladite source de rayonnement,
   - un second support physique agencé pour recevoir un patient,
   dans lequel le premier support physique et le second support physique sont agencés pour être déplacés l'un par rapport à l'autre,
   et dans lequel l'unité de traitement (104) exécute le code de programme utilisable par ordinateur pour
   - trouver (30) le sous-ensemble le plus creux dudit sous-ensemble de tirs individuels de doses pondérés de poids non nuls, pour satisfaire à ladite une ou auxdites plusieurs contraintes de dose (20) chaque fois que le premier support physique est déplacé par rapport au second support physique et/ou chaque fois que le second support physique est déplacé par rapport au premier support physique.

6. Système selon l'une des revendications 1 à 5, dans lequel l'unité de traitement (104) exécute le code de programme utilisable par ordinateur pour trouver (30) le sous-ensemble le plus creux de tirs individuels de doses, de manière à satisfaire à ladite une ou auxdites plusieurs contraintes de dose (20) chaque fois qu'un patient et/ou un organe et/ou une partie d'un organe du patient bouge, et/ou modifie sa forme et/ou change ses dimensions.

7. Système selon l'une des revendications 1 à 5, connectable avec des capteurs pour capturer la cible d'irradiation pendant le traitement, détecter des changements dans la géométrie et/ou la forme et/ou la position et/ou la position et/ou les dimensions de la zone cible, et mettre à jour une configuration cible en conséquence, dans lequel l'unité de traitement (104) exécute le code de programme utilisable par ordinateur pour

   - trouver (30) le sous-ensemble le plus creux de tirs individuels de dose, pour satisfaire ladite ou lesdites contrainte(s) de dose (20) lors desdits changements, sur la base de ladite configuration cible mise à jour.

8. Système selon l'une des revendications 1 à 5, dans lequel l'unité de traitement (104) exécute le code de programme utilisable par ordinateur pour

   - trouver (30) le sous-ensemble le plus creux de tirs individuels de dose, pour satisfaire ladite ou lesdites contrainte(s) de dose (20) chaque fois que la distribution de dose réelle chez le patient à un moment donné est différente de la distribution de dose prévue.

9. Système selon l'une des revendications 1 à 7, dans lequel le nombre de poids non nuls ($s_j$) est au moins $1/100^e$ du nombre de tirs individuels de doses ($a^j$) pré-calculés.

10. Système selon l'une des revendications 1 à 9, dans lequel l'unité de traitement (104) exécute le code de programme utilisable par ordinateur pour

   - minimiser une norme L1 pondérée du vecteur de poids tout en satisfaisant lesdites contraintes de dose (20), pour obtenir un sous-ensemble optimal de tirs individuels de doses.

11. Système selon l'une des revendications 1 à 10, dans lequel l'unité de traitement (104) exécute le code de programme utilisable par ordinateur pour

   - minimiser une norme L2 pondérée du vecteur de poids tout en satisfaisant lesdites contraintes de dose (20), pour obtenir un sous-ensemble optimal de tirs individuels de doses.

12. Système selon l'une des revendications 1 à 11, dans lequel l'unité de traitement (104) exécute le code de programme utilisable par ordinateur pour

   - localiser chaque tir individuel de dose ($a^j$) à un emplacement d'une grille tridimensionnelle (G).

13. Système selon l'une des revendications 1 à 12, dans lequel l'unité de traitement (104) est configurée pour exécuter le code de programme utilisable par ordinateur en temps réel.

14. Système selon l'une des revendications 1 à 13, dans lequel ladite ou lesdites plusieurs contrainte(s) de dose (20) compren(d)nent des contraintes de dose appliquées à la zone cible (T) et/ou à d'autres zones telles que des structures sensibles (R) à protéger d'un rayonnement à trop forte dose.

15. Système selon l'une des revendications 1 à 14, dans lequel l'unité de traitement (104) est configurée pour exécuter le code de programme utilisable par ordinateur afin de

   - prendre en compte les propriétés physiques de l'anatomie du patient pendant le pré-calcul du dictionnaire.

16. Système selon l'une des revendications 1 à 15, dans lequel ledit critère d'optimisation (50) comprenant la réduction au minimum du temps de traitement.

17. Système selon l'une des revendications 1 à 16, dans lequel la source de rayonnement est un accélérateur linéaire.

18. Système selon l'une des revendications 1 à 17, dans lequel la source de rayonnement est une source de cobalt ou un faisceau de protons.

19. Produit-programme informatique, comprenant :

   un support tangible utilisable par ordinateur comprenant un code de programme utilisable par ordinateur pour un système de planification inverse de traitement par rayonnement comprenant une source de rayonnement configurée pour délivrer des tirs de doses individuels (aj), chaque tir de dose individuel ayant un emplacement et un angle d'incidence prédéterminés à l'intérieur et/ou à l'extérieur d'une zone cible, une dimension et une forme, **caractérisé en ce que** le code de programme utilisable par ordinateur est utilisé pour

      - pré-calculer (10) un dictionnaire composé d'un ensemble de tirs individuels de doses possibles ($a^j$) comprenant, pour chaque tir individuel de dose, son emplacement, son angle d'incidence, sa dimension et sa forme,
      - associer (40) un poids ($s_j$) à chaque tir individuel de dose ($a^j$), sur la base d'une ou plusieurs contrainte(s) de dose (20), de manière à obtenir des des tirs individuels de doses pondérés
      dans lequel l'unité de traitement (104) exécute le code de programme utilisable par ordinateur pour

         - appliquer un critère d'optimisation convexe (50), et
         - trouver (30) le sous-ensemble le plus creux desdits tirs individuels de doses pondérés de poids non nuls pour satisfaire à une ou plusieurs contraintes de dose (20).

100

104    106    108

102

Fig.1

110    112    114

118
116

10

20

30

40

Fig.2    50

Fig.3

Fig.4

Fig.5a

Fig.5b

Fig.5c

Fig.6

Fig.8

Fig.7a

Fig.7b

Fig.7c

**EP 3 157 626 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2011085643 A